# EUROPEAN PATENT APPLICATION

(11) **EP 4 678 177 A1**
(43) Date of publication of application: **14.01.2026**
(21) Application number: 24767064.9
(22) Date of filing: 01.03.2024
(51) Int. Cl.: A61K 31/7105, A61K 31/7115, A61K 39/12, A61P 31/14, A61P 37/04

(54) **ARENAVIRUS VACCINE**

(30) Priority: 03.03.2023 JP 2023032739
(71) Applicant: The University of Osaka, Osaka 565-0871 (JP)
(72) Inventor: IWASAKI, Masaharu, Suita-shi, Osaka 565-0871 (JP); HASHIZUME, Mei, Suita-shi, Osaka 565-0871 (JP)
(74) Representative: Marks & Clerk LLP
(86) International application number: PCT/JP2024/007794
(87) International publication number: WO 2024/185696

(57) **Abstract**

Provided is a new vaccine against mammal arenavirus infections. The vaccine comprises lipid nanoparticles containing mRNA encoding a glycoprotein precursor (GPC) or a nucleoprotein (NP) of arenavirus.

## Description

### Technical Field

The present invention relates to an arenavirus vaccine.

### Background Art

Among mammalian arenaviruses, there are multiple pathogens that cause lethal viral hemorrhagic fever in humans. Among such pathogens, Lassa virus, which causes Lassa fever, has the most significant impact on humans. Approximately 900,000 people are infected with Lassa virus in West Africa every year, and the fatality rate of patients who needed to be hospitalized is about 15%, which is very high. Despite being a highly critical human pathogen, there are no established methods for preventing or treating Lassa virus. It is known that virus-specific cellular immunity plays an important role in controlling Lassa virus. Until now, live-attenuated vaccines capable of conferring long-term cellular and humoral immunity with a single dose have been central to the development of Lassa virus vaccines. Several live-attenuated Lassa virus vaccine candidates have been confirmed to have efficacy and safety in animal models infected with Lassa virus. Among such vaccine candidates, a recombinant vesicular stomatitis virus (VSV) vaccine that expresses a Lassa virus glycoprotein has entered phase I clinical trials. However, as a characteristic of live-attenuated vaccines, there is a safety risk for patients with immunodeficiency. In particular, in areas where Lassa virus is endemic, there are also potential HIV-infected individuals. Due to these factors, there has been a demand for the development of a new vaccine against mammalian arenavirus infections.

In recent years, mRNA vaccines that express the SARS-CoV-2 spike protein (SARS2-S-mRNA-LNP) have been used worldwide against COVID-19, which caused a pandemic, and have shown high efficacy (Patent Literature (PTL) 1 and Patent Literature (PTL) 2). Interestingly, SARS2-S-mRNA-LNP has been revealed to induce SARS2-specific CD8⁺ T-cell response as well as a neutralizing antibody, which suggests that not only the neutralizing antibody but also virus-specific cellular immunity contribute to the prevention of onset of the disease and prevention of aggravation of the disease.

### Citation List

### Patent Literature

PTL 1: US2020/0197510A
PTL 2: JP2019-216733A

### Summary of Invention

### Technical Problem

An object of the present invention is to provide a novel vaccine against mammalian arenavirus infections.

### Solution to Problem

To overcome the above object, the present inventors carried out intensive research. As a result, the present inventors found that an mRNA vaccine that expresses a glycoprotein precursor (GPC) or a nucleoprotein (NP) of arenavirus can prevent and/or treat mammalian arenavirus infections. The present invention has been accomplished as a result of further research based on this finding. The present invention includes the following embodiments.

### Item 1.

A vaccine comprising lipid nanoparticles each containing mRNA encoding a glycoprotein precursor (GPC) of arenavirus or mRNA encoding a nucleoprotein (NP) of arenavirus, or both.

### Item 2.

The vaccine according to Item 1, wherein the GPC and the NP are of the same or different origins and are derived from Lassa virus or lymphocytic choriomeningitis virus.

### Item 3.

The vaccine according to Item 1 or 2, wherein the GPC and the NP are of a wild type.

### Item 4.

The vaccine according to any one of Items 1 to 3, comprising lipid nanoparticles in which the mRNA encoding the GPC of arenavirus and the mRNA encoding the NP of arenavirus are encapsulated together in the same nanoparticle or individually encapsulated in separate nanoparticles.

### Item 5.

The vaccine according to any one of Items 1 to 4, wherein the mRNA encoding the GPC of arenavirus or the mRNA encoding the NP of arenavirus further comprises a 5' cap structure, a 5' untranslated region (UTR), a 3' UTR, and a 3' polyadenylated tail.

### Item 6.

The vaccine according to Item 5, wherein the 5' UTR and the 3' UTR are derived from β-globin or α-globin.

### Item 7.

The vaccine according to Item 5 or 6, wherein the 3' polyadenylated tail is 200 nucleotides or less in length.

### Item 8.

The vaccine according to any one of Items 1 to 7, wherein uridine contained in the mRNA is 5-methoxyuridine.

### Item 9.

The vaccine according to any one of Items 1 to 8, wherein the lipid nanoparticles have a number average particle size of 70 nm to 130 nm.

### Item 10.

The vaccine according to any one of Items 1 to 9 for intravenous or intramuscular administration.

### Item 11.

The vaccine according to any one of Items 1 to 10 for use in administration to a subject at risk of arenavirus infection.

### Item 12.

The vaccine according to Item 11, wherein the arenavirus is Lassa virus and/or lymphocytic choriomeningitis virus.

### Item 13.

The vaccine according to any one of Items 1 to 12, which is for use in administration in an amount such that the mRNA content per dose of the vaccine is 0.1 µg to 200 µg.

### Item 14.

A method for preventing an arenavirus infection, comprising administering a vaccine comprising lipid nanoparticles each containing mRNA encoding a glycoprotein precursor (GPC) of arenavirus or mRNA encoding a nucleoprotein (NP) of arenavirus, or both.

### Item 15.

Use of lipid nanoparticles each containing mRNA encoding a glycoprotein precursor (GPC) of arenavirus or mRNA encoding a nucleoprotein (NP) of arenavirus, or both in the production of a vaccine.

### Advantageous Effects of Invention

According to the present invention, a novel vaccine against a mammalian arenavirus infection can be provided.

### Brief Description of Drawings

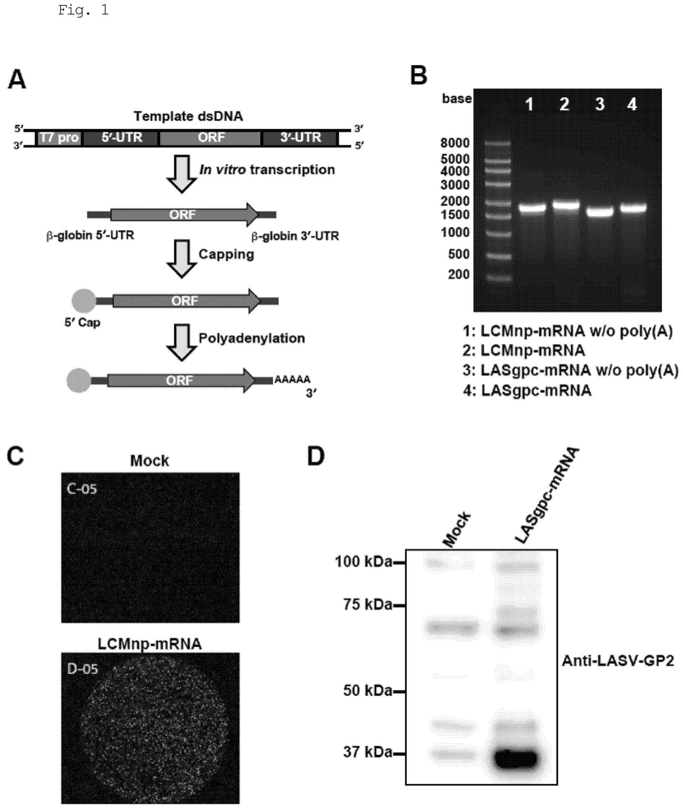

Fig. 1 is diagrams showing the synthesis of IVT-mRNA that expresses LASgpc or LCMnp. (A): a schematic diagram showing the synthesis of IVT-mRNA that encodes the ORF of a viral protein. Using a PCR-amplified DNA fragment as a template, naked RNA comprising the 5'-UTR of human β-globin, the ORF of a viral protein, and the 3'-UTR of human β-globin was synthesized by in vitro transcription. A 5' cap structure was added to the naked RNA, followed by polyadenylation at the 3' end. (B): IVT-mRNA with or without (w/o) poly A tail was subjected to agarose gel electrophoresis. (C) and (D): 200 ng of LCMnp-mRNA (C) or LASgpc-mRNA (D) was introduced into HEK293T cells, whereas Opti-MEM was introduced into HEK293T cells as a mock. (C): 24 hours after introduction, LCMnp expression in fixed cells was examined by indirect immunofluorescence using a monoclonal antibody against LCMnp. (D): LASgpc in the cell lysate from which insoluble components had been removed was examined by western blotting using a polyclonal antibody against LASV GP2.

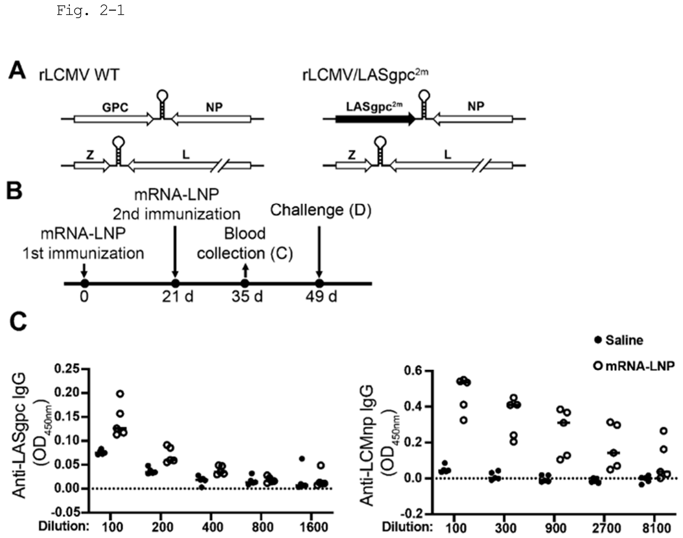

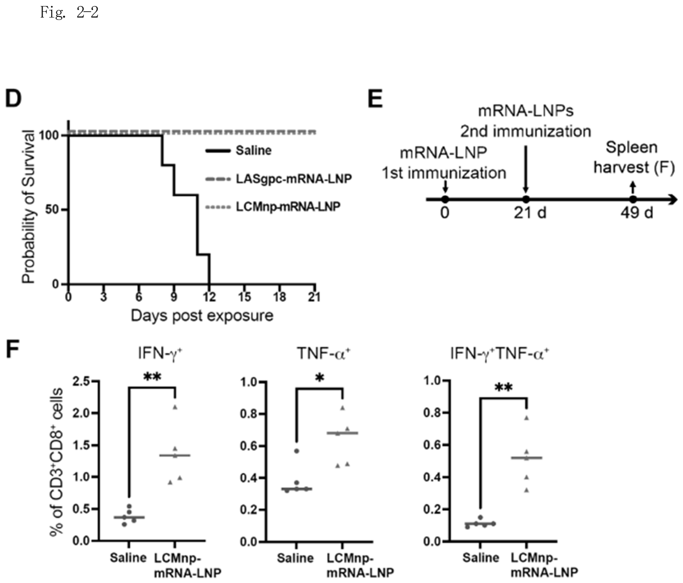

Fig. 2-1 is diagrams showing the induction of protective immunity by intravenous administration of mRNA-LNP vaccine in C57BL/6 mice. (A): a schematic diagram of an rLCMV/LASgpc^{2m} genome structure. (B): a schematic diagram of experiments (C) and (D). (C): LASgpc-mRNA-LNP, LCMnp-mRNA-LNP, or physiological saline was intravenously administered to 6-week-old C57BL/6 mice (n = 5) twice, 21 days apart. On day 14 after the second administration, the anti-LASgpc IgG (left) and anti-LCMnp IgG (right) levels in plasma were measured.
Fig. 2-2 is diagrams showing the induction of protective immunity by intravenous administration of mRNA-LNP vaccine in C57BL/6 mice. (D): On day 28 after the second administration, C57BL/6 mice were inoculated intracranially with rLCMV/LASgpc^{2m}, and their survival was monitored daily. (E): a schematic diagram of experiment (F). (F): LCMnp-mRNA-LNP or physiological saline was intravenously administered to six-week-old C57BL/6 mice (n = 5) twice, 21 days apart. On day 28 after the second administration, cytokine production by spleen CD3⁺CD8⁺ T cells that respond specifically to NP396 peptide was examined by flow cytometry. Data are presented as mean ± SD. **p < 0.01, *p < 0.05.

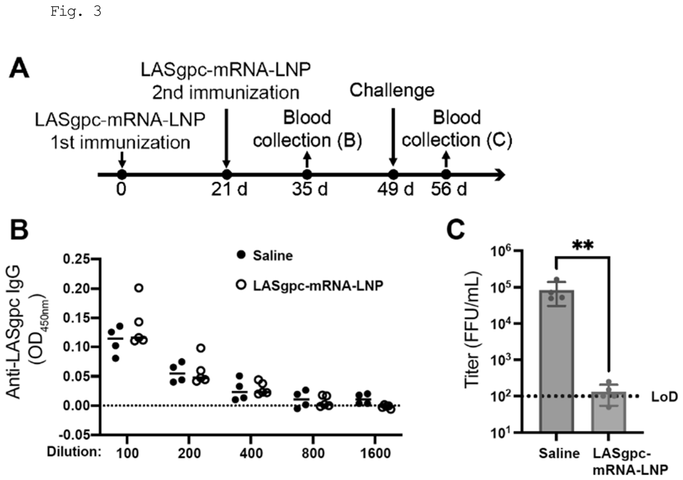

Fig. 3 is diagrams showing the induction of protective immunity by intramuscular administration of LASgpc-mRNA-LNP vaccine into C57BL/6 mice. (A): a schematic diagram of the experiment. LASgpc-mRNA-LNP (n = 5) or physiological saline (n = 4) was intramuscularly administered to 6-week-old C57BL/6 mice twice, 21 days apart. (B): The anti-LASgpc IgG level in plasma was measured 14 days after the second administration. (C): On day 28 after the second administration, C57BL/6 mice were inoculated intravenously with rLCMV/LASgpc^{2m} and the viral titer in plasma was measured. Data are presented as mean ± SD. "LoD" stands for "low limit of detection" (detection limit). **p < 0.01.

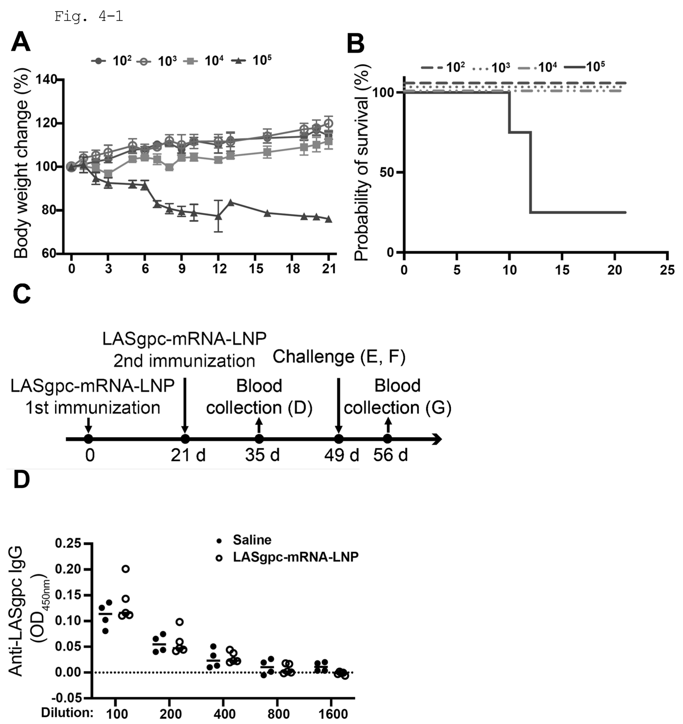

Fig. 4-1 is diagrams showing the induction of virus-specific immunity by intramuscular administration of LASgpc-mRNA-LNP vaccine in CBA mice and protective immunity effects against lethal rLCMV/LASgpc^{2m} exposure. (A) and (B): 8-week-old CBA mice (n = 4) were inoculated intravenously with rLCMV/LASgpc^{2m}. Their body weight changes (A) and survival rates (B) were monitored daily. (C): a schematic diagram of experiments (D) to (G). (D): LASgpc-mRNA-LNP or physiological saline was intramuscularly administered to 6-week-old CBA mice (n = 5) twice, 21 days apart. The anti-LASgpc IgG level in plasma was measured 14 days after the second administration.

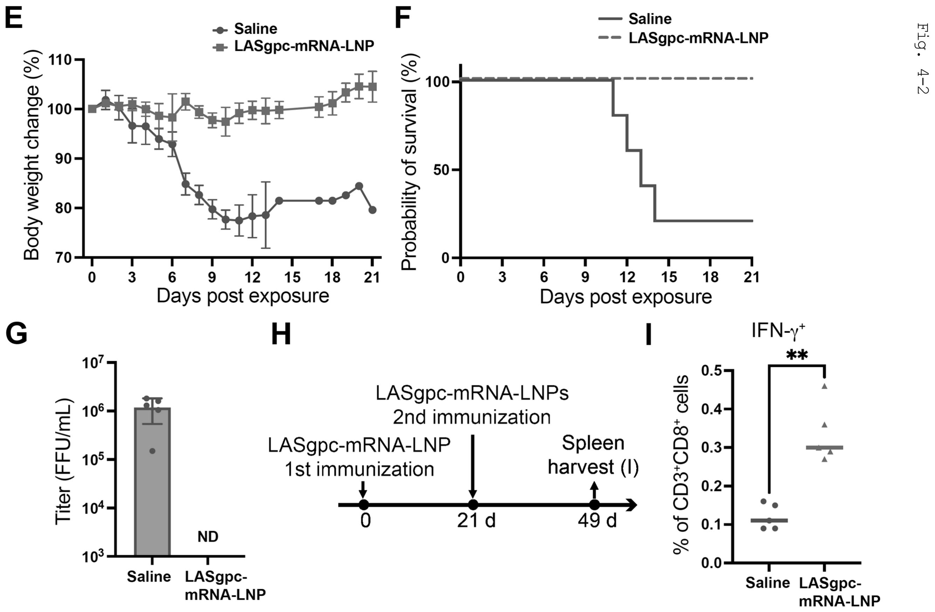

Fig. 4-2 is diagrams showing the induction of virus-specific immunity and protective immunity effect against lethal rLCMV/LASgpc^{2m} exposure by intramuscular administration of LASgpc-mRNA-LNP vaccine into CBA mice. (E) and (F): CBA mice were inoculated intravenously with rLCMV/LASgpc^{2m} on day 28 after the second administration. Their body weight changes (E) and survival rates (F) were monitored daily. (G): The virus titer in plasma was measured on day 7 after virus exposure. Data are presented as mean ± SD. "ND" stands for "not detected" (no detection). (H): a schematic diagram of experiment (I). (I): LASgpc-mRNA-LNP or physiological saline was intramuscularly administered to 6-week-old CBA mice (5 mice per group) twice, 21 days apart. On day 28 after the second administration, cytokine production by spleen CD3⁺CD8⁺ T cells that specifically respond to LASgpc peptide cocktail was examined by flow cytometry. **p < 0.01.

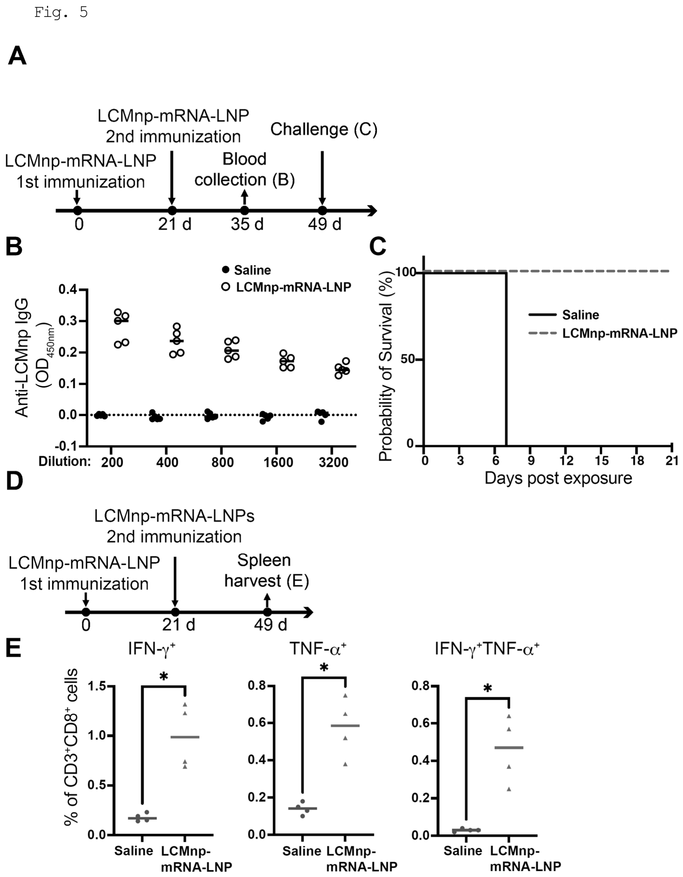

Fig. 5 is diagrams showing virus-specific immunization and protective immunity effects against virus exposure that causes lethal hemorrhagic conditions achieved by intramuscular administration of LCMnp-mRNA-LNP vaccine in FVB mice. (A): a schematic diagram of experiments (B) and (C). (B): LCMnp-mRNA-LNP or physiological saline was intramuscularly administered to 6-week-old FVB mice (5 mice per group) twice, 21 days apart. The anti-LCMnp IgG level in plasma was measured on day 14 after the second administration. (C): On day 28 after the second administration, FVB mice were inoculated intravenously with wild-type rLCMV, and their survival rates were monitored daily. (D): a schematic diagram of experiment (E). (E): LCMnp-mRNA-LNP or physiological saline was intramuscularly administered to 6-week-old FVB mice (4 mice per group) twice, 21 days apart. On day 28 after the second administration, cytokine production by spleen CD3⁺CD8⁺ T cells that specifically respond to NP118 peptide was examined by flow cytometry. *p < 0.05.

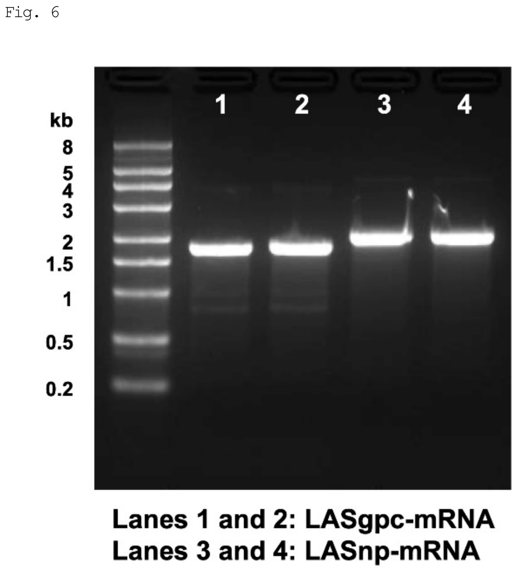

Fig. 6 is a photograph of agarose gel electrophoresis of IVT-mRNA that expresses LASgpc or LASnp produced by the method described in Example 11.

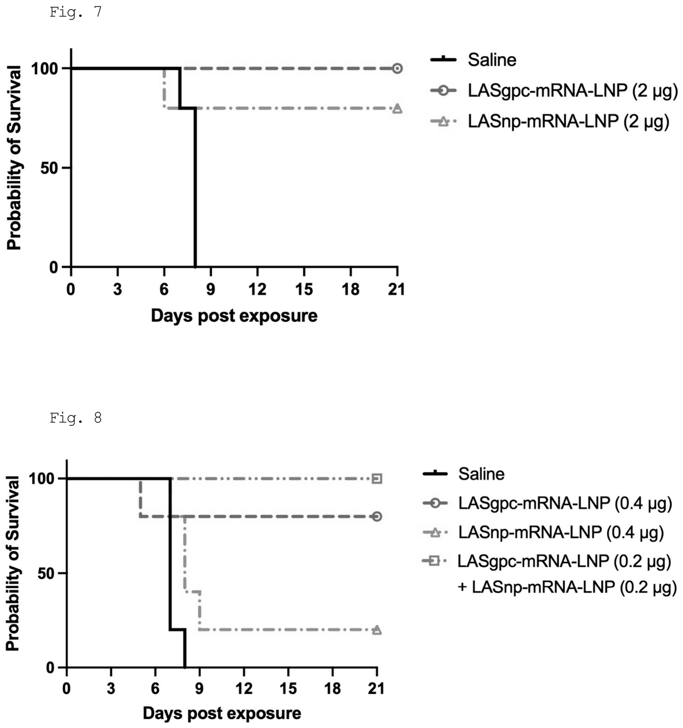

Fig. 7 is a graph showing the results of monitoring daily survival of CBA mice to which physiological saline or each vaccine was intramuscularly administered in the test of Example 11.
Fig. 8 is a graph showing the results of monitoring daily survival of CBA mice to which either physiological saline or one of the vaccines was intramuscularly administered in the test of Example 12.

### Description of Embodiments

### 1. Vaccine of the present invention

The vaccine of the present invention comprises lipid nanoparticles containing mRNA encoding a glycoprotein precursor (GPC) or mRNA encoding a nucleoprotein (NP) of arenavirus. The vaccine of the present invention may comprise lipid nanoparticles in which the mRNA encoding the GPC and the mRNA encoding the NP are encapsulated together in the same nanoparticle or individually present in separate nanoparticles. In this case, significantly enhanced immunity induction is achieved, as compared to vaccines comprising nanoparticles containing only mRNA encoding the GPC or vaccines comprising nanoparticles containing only mRNA encoding the NP.

The GPC or NP encoded by mRNA encapsulated in the lipid nanoparticles contained in the vaccine of the present invention is a protein derived from an arenavirus or a protein fragment thereof.

The mRNA encoding GPC and the mRNA encoding NP are preferably of the same or different origins and are derived from Lassa virus or lymphocytic choriomeningitis virus. mRNA encoding GPC derived from Lassa virus or mRNA encoding NP derived from lymphocytic choriomeningitis virus is more preferable.

The mRNA encoding GPC or the mRNA encoding NP is not particularly limited in structure as long as it is translatable. The mRNA preferably has a structure similar to that of the open reading frame (ORF) of natural mRNA; i.e., it is preferably of a natural type. Examples of the "structure similar to that of the ORF of natural mRNA" include mRNA in which uridine, which is a constituent of mRNA, is completely substituted with chemically modified uridine, as is described below.

The mRNA encoding GPC or the mRNA encoding NP may further comprise a 5' cap structure, a 5' untranslated region (UTR), 3' UTR, and a 3' polyadenyl chain in addition to the above structure.

The 5' cap structure is preferably of the Cap 1 type.

The 5' UTR and the 3' UTR are preferably derived from human β-globin or human α-globin.

The 3' polyadenyl tail is preferably 200 nucleotides or less in length, and more preferably 150 nucleotides or less in length.

In the mRNA encapsulated in the lipid nanoparticles contained in the vaccine of the present invention, uridine is preferably completely substituted with chemically modified uridine from the perspective of, for example, improving mRNA stability and reducing translation inhibition caused by natural immunity activation. Examples of chemically modified uridine include 5-methoxyuridine, pseudouridine, N1-methyl, and 2-thiouridine. It is particularly preferable for all the uridine nucleotides to be replaced by 5-methoxyuridine.

The mRNA encapsulated in the lipid nanoparticles contained in the vaccine of the present invention can be designed by a conventional method using generally used molecular and cellular tools, and can be produced by various known methods.

The number average particle size of the lipid nanoparticles contained in the vaccine of the present invention is preferably 70 nm to 130 nm, more preferably 80 nm to 120 nm, and even more preferably 90 nm to 110 nm.

The "average particle size of the lipid nanoparticles" refers to the number-average particle diameter as determined by dynamic light scattering (DLS), unless otherwise specified. The measurement by dynamic light scattering can be performed, for example, by using commercially available DLS equipment.

The lipid nanoparticles contained in the vaccine of the present invention are composed of, for example, 30 to 50 mol% of ionizable cationic lipid (pH-sensitive lipid), 35 to 50 mol% of sterol, 0.5 to 3 mol% of PEG-modified lipid, and 10 mol% or less of neutral phospholipid, based on 100 mol% of the lipid nanoparticles.

Examples of ionizable cationic lipids include dilinoleyl-methyl-4-dimethylaminobutyrate (DLin-MC3-DMA), 1,2-dioleoyl-3-dimethylaminopropane (DODMA), [(4-hydroxybutyl)azanediyl]bis(hexane-6,1-diyl)bis(2-hexyldecanoate) (ALC-0315), heptadecan-9-yl 8-((2-hydroxyethyl) (6-oxo-6-(undecyloxy)hexyl)amino)octanoate (SM-102), 2,2-dilinoleyl-4-dimethylaminoethyl-[1,3]-dioxolane (DLin-KC2-DMA), and di((Z)-nona-2-en-1-yl) 9-((4-(dimethylamino)butanoyl)oxy)heptadecanedioate (L319).

Examples of sterols include cholesterol, fecosterol, sitosterol, ergosterol, campesterol, stigmasterol, brassicasterol, tomatidine, tomatine, ursolic acid, and alpha-tocopherol.

Examples of PEG-modified lipids include 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-distearylglycerol (PEG-DSG), PEG-dipalmitoyl, PEG-dioleoyl, PEG-distearyl, PEG-diacylglycamide (PEG-DAG), PEG-dipalmitoylphosphatidylethanolamine (PEG-DPPE), and PEG-1,2-dimyristyloxypropyl-3-amine (PEG-c-DMA).

Examples of neutral phospholipids include 5-heptadecylbenzene-1,3-diol (resorcinol), dipalmitoylphosphatidylcholine (DPPC), distearoylphosphatidylcholine (DSPC), dioleoylphosphatidylcholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauroylphosphatidylcholine (DLPC), dimyristoylphosphatidylcholine (DMPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1,2-diarachidoyl-sn-glycero-3-phosphocholine (DBPC), 1-stearoyl-2-palmitoylphosphatidylcholine (SPPC), 1,2-dieicosenoyl-sn-glycero-3-phosphocholine (DEPC), palmitoyloleoylphosphatidylcholine (POPC), lysophosphatidylcholine, dioleoylphosphatidylethanolamine (DOPE), dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, and combinations thereof.

The method for producing the lipid nanoparticles contained in the vaccine of the present invention is not particularly limited. Any method available to those skilled in the art can be used. For example, a commercially available device for producing nanoparticles can be used.

The form of the lipid nanoparticles contained in the vaccine of the present invention is not particularly limited. Examples include unilamellar liposomes, multilamellar liposomes, spherical micelles, and amorphous lamellar structures.

The lipid nanoparticles contained in the vaccine of the present invention may contain any other components, in addition to the mRNA described above, in the particles. Examples of such other components include adjuvants, stabilizers, and preservatives.

Examples of adjuvants include aluminum salts. Examples of aluminum salts include aluminum hydroxide and aluminum phosphide.

Examples of stabilizers include refined white sugar, sodium chloride, potassium chloride, magnesium chloride, disodium hydrogen phosphate dihydrate, potassium dihydrogen phosphate, sodium acetate hydrate, glacial acetic acid, tris(hydroxymethyl)aminomethane (also known as "trometamol"), trometamol hydrochloride, L-histidine, L-histidine hydrochloride hydrate, anhydrous ethanol, and polyoxyethylene sorbitan oleate (also known as "polysorbate 80").

Examples of preservatives include sodium ethylmercurithiosalicylate (also known as "thimerosal") and phenoxyethanol.

The vaccine of the present invention can be applied to arenavirus infections, particularly mammalian arenavirus infections. Examples of diseases caused by mammalian arenavirus infections include Lassa fever; Lujo hemorrhagic fever; South American hemorrhagic fevers, such as Argentine hemorrhagic fever, Bolivian hemorrhagic fever, Brazilian hemorrhagic fever, Venezuelan hemorrhagic fever, and Chapare hemorrhagic fever; and lymphocytic choriomeningitis. Examples of pathogens of mammalian arenavirus infections include Lassa virus, Lujo virus, Junin virus, Machupo virus, Sabia virus, Guanarito virus, Chapare virus, and lymphocytic choriomeningitis virus.

The vaccine of the present invention is administered to a subject at risk of infection with arenavirus. The vaccine of the present invention is preferably administered to a subject at risk of infection with, in particular, Lassa virus and/or lymphocytic choriomeningitis virus. The term "subject" refers to a human or a non-human animal. The "animal" includes mammals, rodents, birds, reptiles, amphibians, fish, and insects. The "non-human animal" refers to a mammal, such as mice, rats, rabbits, monkeys, dogs, cats, sheep, cattle, primates, or pigs.

The method for administering the vaccine of the present invention can be appropriately selected according to the target disease, the condition of the subject, and other conditions. Examples of administration methods include intravenous administration, intramuscular administration, subcutaneous administration, intradermal administration, intranasal administration, oral administration, intralymphatic administration, and transmucosal administration. Among these, the administration method is preferably intravenous administration or intramuscular administration, with intramuscular administration being more preferable.

Dosing frequency and dosing interval of the vaccine of the present invention can be appropriately selected according to the target disease, the condition of the subject, the route of administration, and other conditions. The dosing frequency of the vaccine of the present invention is preferably, for example, at least twice. Further, the dosing interval of the vaccine of the present invention is preferably, for example, at least 3 weeks.

The dose of the vaccine of the present invention can be appropriately selected according to the target disease, the condition of the subject, the route of administration, and other conditions. The dose of the vaccine of the present invention can be appropriately determined according to the age and gender of the subject, the condition, etc. by a method known to those skilled in the art. For example, the vaccine is preferably administered in such an amount that the mRNA content per dose of the vaccine is 0.1 µg to 200 µg, and more preferably to be 5 µg to 50 µg.

### Examples

The present invention is described below in more detail with reference to Examples. However, the present invention is not limited to these Examples.

### Example 1: Preparation of in vitro transcription (IVT) mRNA and confirmation of expression in cultured cells

The mRNA capable of expressing a glycoprotein precursor (GPC) of Lassa virus (LASV) (referred to below as "LASgpc") (which is referred to below as "LASgpc-mRNA") and the mRNA capable of expressing a nucleoprotein (NP) of Lymphocytic choriomeningitis virus (LCMV) (referred to below as "LCMnp") (which is referred to below as "LCMnp-mRNA") were synthetically produced in vitro (Figs. 1A and 1B). The expression of the glycoprotein precursor (GPC) and the nucleoprotein (NP) in cultured cells was confirmed (Figs. 1C and 1D).

In the LASgpc-mRNA and LCMnp-mRNA thus synthesized, uridine was replaced by 5-methoxyuridine, which is a modified uridine, wherein a cap structure was added to the 5' end, and a polyadenyl tail was added to the 3' end (Fig. 1A).

### Synthetic mRNA

DNA fragments composed of a T7 promoter sequence with a GGG sequence at the 3' end, a human β-globin (NCBI Accession ID: NM_00518.5) 5'-UTR sequence, a human β-globin 3'-UTR sequence, and an open reading frame (ORF) of either LASgpc (NCBI Accession ID: NC_004296.1) or LCMnp (NCBI Accession ID: KY514256.1) with a stop codon were amplified by PCR. These DNA fragments were used as templates to generate 5'-capped and 3'-polyadenylated mRNAs using the T7 mScript Standard mRNA Production System (produced by CellScript, Madison, WI, USA), in which uridine-5'-triphosphate was replaced by 5-methoxyuridine-5'-triphosphate (produced by TriLink BioTechnologies). IVT-mRNAs were then purified using a Monarch RNA Cleanup Kit (produced by New England Biolabs), and the quality of purified IVT-mRNAs was assessed by non-denaturing agarose gel electrophoresis using RNA High for Easy Electrophoresis (produced by DynaMarker Laboratory) (Fig. 1B).

### Example 2: Preparation of mRNA-LNP

The LASgpc-mRNA or LCMnp-mRNA synthesized in Example 1 were encapsulated into lipid nanoparticles (LNP) (LASgpc-mRNA encapsulated in LNP is referred to below as "LASgpc-mRNA-LNP"; LCMnp-mRNA encapsulated in LNP is referred to below as "LCMnp-mRNA-LNP"; and these are collectively referred to below as "mRNA-LNP.")

### Preparation of mRNA-LNP

LNP formulations (IVT-mRNA-LNPs) were prepared from GenVoy-ILM and IVT-mRNA using a NanoAssemblr^{®} Ignite microfluidic mixer (produced by Precision Nanosystems). The N/P ratio (the molar ratio of negatively charged phosphates on mRNA to cationic amines on ionizable lipids) was set to 4. The resulting samples were diluted with 1× Formulation Buffer 2 (produced by Precision Nanosystems), concentrated with Amicon Ultra Centrifugal Filters (produced by Millipore), and passed through a 0.2-µm filter according to the manufacturer's recommendations (produced by Precision Nanosystems). Sterile-filtered mRNA-LNPs were stored at 4°C before use. The concentration of IVT-mRNA incorporated into LNPs was determined using a Quant-iT Ribogreen Assay Kit (produced by Thermo Fisher Scientific).

### Example 3: Preparation of recombinant LCMV (rLCMV/LASgpc2m) expressing LASV-GPC

A recombinant LCMV (referred to below as "rLCMV/LASgpc^{2m}") was prepared by replacing LCMV-GPC gene with LASV-GPC gene containing two mutations that are V459K and K461G mutations (Fig. 2-1A).

After inoculation with 10⁶ focus-forming units (FFU) of rLCMV/LASgpc^{2m}, high levels of viremia were confirmed in C57BL/6 mice 7 days. The C57BL/6 mice inoculated intracranially with 10³ FFU of rLCMV/LASgpc^{2m} all died, although their survival time was slightly prolonged as compared to that of the mice inoculated intracranially with wild-type rLCMV (WT rLCMV). These results show that rLCMV/LASgpc^{2m} is usable as a mouse model for LASV infection. Accordingly, in subsequent experiments, protective immunity effects of LASgpc-mRNA-LNP and LCMnp-mRNA-LNP against LASV infection were verified by using rLCMV/LASgpc^{2m}.

### Example 4: Verification of inducibility of protective immunity by intravenous administration of mRNA-LNP vaccine

To verify the possibility to induce protective immunity by LASgpc-mRNA-LNP and LCMnp-mRNA-LNP, LASgpc-mRNA-LNP or LCMnp-mRNA-LNP each containing 10 µg of IVT-mRNA, or physiological saline was administered intravenously to C57BL/6 mice twice, 3 weeks apart (Fig. 2-1B). The LASgpc antibody and LCMnp antibody levels in plasma were measured by enzyme-linked immunosorbent assays (ELISAs) on day 14 after the second administration.

Fig. 2-1C shows the results. LCMnp-specific antibody production was strongly induced in C57BL/6 mice to which LCMnp-mRNA-LNP was administered, whereas substantially no LASgpc-specific antibodies were detected in C57BL/6 mice to which LASgpc-mRNA-LNP was administered.

### Example 5: Verification of protective immunity effects of mRNA-LNP vaccine against lethal virus exposure

To verify protective immunity effects of LASgpc-mRNA-LNP and LCMnp-mRNA-LNP against exposure to a lethal dose of rLCMV/LASgpc^{2m}, either the LASgpc-mRNA-LNP or LCMnp-mRNA-LNP, or physiological saline was intravenously administered to C57BL/6 mice twice, 3 weeks apart, followed by intracranial inoculation with a lethal dose of rLCMV/LASgpc^{2m} on day 28 after the second administration.

Fig. 2-2D shows the results. C57BL/6 mice that received physiological saline died from rLCMV/LASgpc^{2m} infection within 12 days after exposure to the virus. In contrast, all the C57BL/6 mice to which either LASgpc-mRNA-LNP or LCMnp-mRNA-LNP was administered survived without showing apparent clinical signs. These results demonstrate that LASgpc-mRNA-LNP and LCMnp-mRNA-LNP have protective immunity effects against exposure to a lethal dose of rLCMV/LASgpc^{2m}.

Considering that the LASgpc-specific antibody in C57BL/6 mice to which LASgpc-mRNA-LNP was administered was at a significantly low level (Fig. 2-1C) and that LCMnp is an intracellular protein, the results suggest that virus antigen-specific cellular T-cell response plays an important role in protective immunity effects of LASgpc-mRNA-LNP and LCMnp-mRNA-LNP against exposure to rLCMV/LASgpc^{2m} shown in Fig. 2-2D.

### Example 6: Verification of inducibility of virus-specific T-cell response by LCMnp-mRNA-LNP vaccine

To verify the possibility that virus antigen-specific T-cell response plays an important role in the protective immunity effect of LCMnp-mRNA-LNP against exposure to rLCMV/LASgpc^{2m}, red-blood-cell-free splenocytes obtained from C57BL/6 mice to which LCMnp-mRNA-LNP or physiological saline was intravenously administered twice (Fig. 2-2E) were cultured in the presence of H-2d-restricted LCMnp T-cell epitope peptide (NP396), and intracellular cytokine expression was examined by flow cytometry.

Fig. 2-2F shows the results. In spleen cells obtained from mice to which LCMnp-mRNA-LNP was administered, significant increases in IFN-γ positive, TNF-α positive, and IFN-γ/TNF-α double-positive CD8⁺ T cells were observed as compared to those of spleen cells obtained from mice that received physiological saline. These results show that virus antigen-specific cellular T-cell response is involved in the protective immunity effect of LCMnp-mRNA-LNP against exposure to rLCMV/LASgpc^{2m}. The spleen cells obtained from mice to which LCMnp-mRNA-LNP was administered were also shown to have multifunctional characteristics as antiviral CD8⁺ T cells.

### Example 7: Verification of inducibility of virus-specific immunity and protective immunity effects against lethal virus exposure by intramuscular administration of LASgpc-mRNA-LNP vaccine in C57BL/6 mice

Whether the protective immunity effect induced by intravenous administration of LASgpc-mRNA-LNP, as demonstrated in Example 5, could also be confirmed by intramuscular administration of LASgpc-mRNA-LNP was verified. For this verification, either LASgpc-mRNA-LNP containing 2 µg of IVT-mRNA or physiological saline was intramuscularly administered to C57BL/6 mice twice, 3 weeks apart. Blood was collected from the mice to investigate the possibility to induce protective immunity and protective immunity effects against virus exposure (Fig. 3A).

Fig. 3B shows the results of verifying the inducibility of virus-specific immunity by intramuscular administration of the LASgpc-mRNA-LNP vaccine. As in the results demonstrated in Example 4 (Fig. 2-1C), substantially no LASgpc-specific antibody was detected in plasma collected from C57BL/6 mice on day 14 after the second administration of LASgpc-mRNA-LNP or physiological saline.

Fig. 3C shows the results of verifying protective immunity effects against virus exposure by intramuscular administration of LASgpc-mRNA-LNP vaccine in C57BL/6 mice. On day 28 after the second administration of LASgpc-mRNA-LNP or physiological saline, the mice were inoculated intravenously with 10⁶ FFU of rLCMV/LASgpc^{2m}, and the virus titer in plasma was measured on day 7 after the inoculation. The viral titer in the plasma of C57BL/6 mice to which LASgpc-mRNA-LNP was administered intramuscularly was significantly reduced to nearly the detection limit (10² FFU/mL), whereas the viral titer in the plasma of C57BL/6 mice that received physiological saline intramuscularly was high. These results show that intramuscular administration of LASgpc-mRNA-LNP provides protective immunity effects against virus exposure in C57BL/6 mice.

### Example 8: Verification of inducibility of virus-specific immunity and protective immunity effects against lethal virus exposure by intramuscular administration of LASgpc-mRNA-LNP vaccine in CBA mice

CBA mice are known to be more highly susceptible to LCMV than C57BL/6 mice. Whether the induction of protective immunity in C57BL/6 mice by intramuscular administration of LASgpc-mRNA-LNP, as demonstrated in Example 6, would also occur in CBA mice was verified.

To determine the appropriate exposure dose of rLCMV/LASgpc^{2m} to CBA mice, various amounts of rLCMV/LASgpc^{2m} (10² to 10⁵ FFU per individual) were inoculated intravenously into CBA mice, and clinical signs, body weight changes, and survival were monitored.

Inoculation of 10⁵ FFU of rLCMV/LASgpc^{2m} caused continuous body weight loss until the end of the experiment (on day 21 after the virus inoculation) (Fig. 4-1A), and three-quarters of the mice reached the criteria for euthanasia (Fig. 4-1B). In contrast, inoculation of 10⁴ FFU or less of rLCMV/LASgpc^{2m} did not cause body weight loss. Therefore, in the following experiments intended to validate LASgpc-mRNA-LNP vaccine efficacy in CBA mice, 10⁵ FFU of rLCMV/LASgpc^{2m} was decided to be inoculated into CBA mice.

LASgpc-mRNA-LNP containing 2 µg of IVT-mRNA or physiological saline was intramuscularly administered to CBA mice twice, 3 weeks apart (Fig. 4-1C). The LASgpc-specific antibody level in plasma collected from the CBA mice on day 14 after the second administration was checked (4-1D). Substantially no LASgpc-specific antibody was detected from the plasma of the CBA mice.

Figs. 4-2E to 4-2G show the results of verifying protective immunity effects against lethal virus exposure by intramuscular administration of the LASgpc-mRNA-LNP vaccine in CBA mice. On day 28 after the second administration of LASgpc-mRNA-LNP or physiological saline, 10⁵ FFU of rLCMV/LASgpc^{2m} was inoculated intravenously, and blood was collected from the CBA mice on day 7 after the inoculation (Fig. 4-1C). All physiological saline-treated CBA mice showed significant body weight loss and four-fifths of the individuals eventually died or reached the criteria for euthanasia, whereas all CBA mice to which LASgpc-mRNA-LNP was administered survived without showing any apparent clinical signs or body weight loss (Fig. 4-2E and Fig. 4-2F). Further, the viral titer in the plasma derived from CBA mice that received LASgpc-mRNA-LNP was suppressed to below the minimum detection level (10³ FFU/mL), as compared to the high viral titer of approximately 10⁶ FFU/mL in the plasma derived from CBA mice that received physiological saline (Fig. 4-2G). These results showed that intramuscular administration of LASgpc-mRNA-LNP vaccine provides protective immunity effects against lethal virus exposure in CBA mice.

The intramuscular administration of the LASgpc-mRNA-LNP vaccine in CBA mice demonstrated protective immunity effects against lethal virus exposure, whereas no LASgpc-specific antibody response was observed. These results suggest that virus antigen-specific T-cell response is involved in protective immunity effects of LASgpc-mRNA-LNP against rLCMV/LASgpc^{2m} exposure.

### Example 9: Verification of the inducibility of virus antigen-specific T-cell response by LASgpc-mRNA-LNP vaccine in CBA mice

To investigate whether LASgpc-specific CD8⁺ T-cell response is involved in the protective immunity effect of LASgpc-mRNA-LNP vaccine against lethal virus exposure, red-blood-cell-free splenocytes obtained from CBA mice to which LASgpc-mRNA-LNP or physiological saline was intramuscularly administered twice (Fig. 4-2H) were stimulated with an LASgpc peptide cocktail, and intracellular cytokine expression was examined by flow cytometry.

Fig. 4-2I shows the results. In spleen cells obtained from mice that received LASgpc-mRNA-LNP, a significant increase in IFN-γ positive CD8⁺ T cells was observed as compared to spleen cells obtained from mice that received physiological saline. These results showed that LASgpc-specific T-cell response is associated with protective immunity effects of LASgpc-mRNA-LNP against exposure to rLCMV/LASgpc^{2m}.

### Example 10: Verification of humoral and cellular immunity induction and immunity protective effect against virus exposure that causes lethal hemorrhagic condition by intramuscular administration of LCMnp-mRNA-LNP vaccine in FVB mice

To investigate whether an mRNA-LNP vaccine also shows protective immunity effects against Lassa fever-like symptoms, the following experiment was performed using LCMV-infected mouse models (FVB mice) that exhibited pathology similar to Lassa fever.

LCMnp-mRNA-LNP containing 2 µg of IVT-mRNA or physiological saline was intramuscularly administered to FVB mice twice, 3 weeks apart. Blood was collected from the mice on day 14 after the second administration (Fig. 5A). FVB mice that received LCMnp-mRNA-LNP produced high levels of LCMnp-specific antibody (Fig. 5B). This result showed that intramuscular administration of LCMnp-mRNA-LNP vaccine induces humoral immunity.

On day 28 after the second administration of LCMnp-mRNA-LNP or physiological saline, a normally lethal dose of WT rLCMV was intravenously administered (Fig. 5A), and clinical signs and survival were monitored. All FVB mice that received LCMnp-mRNA-LNP survived without showing apparent clinical signs, whereas FVB mice that received physiological saline died within 8 days after inoculation with WT rLCMV (Fig. 5C). These results demonstrate that intramuscular administration of the LCMnp-mRNA-LNP vaccine provides protective effects against virus exposure that causes lethal hemorrhagic disease.

To investigate the involvement of virus-specific T-cell response in the protective effect against lethal hemorrhagic disease-causing virus exposure by intramuscular administration of LCMnp-mRNA-LNP vaccine, red-blood-cell-free splenocytes obtained from FVB mice to which LCMnp-mRNA-LNP or physiological saline was intramuscularly administered twice (Fig. 5D) were stimulated with H-2q-restricted LCMnp-specific T-cell epitope peptide (NP118), and intracellular cytokine expression was examined by flow cytometry. As a result, significant increases in IFN-γ positive, TNF-α positive, and IFN-γ/TNF-α double-positive CD8⁺ T cells were observed in FVB mice that received LCMnp-mRNA-LNP, as compared to those in FVB mice that received physiological saline (Fig. 5E). These results show that intramuscular administration of LCMnp-mRNA-LNP vaccine induces cellular immunity.

### Example 11: Verification of protective effect by intramuscular administration of LASnp-mRNA-LNP vaccine in CBA mice

Whether intramuscular administration of an mRNA-LNP vaccine (LASnp-mRNA-LNP) using LASgpc-mRNA or mRNA expressing the NP of Lassa virus (referred to below as "LASnp") (LASnp-mRNA) could induce protective immunity in CBA mice was verified.

The ORF of LASgpc or LASnp was cloned into a Linearized Template Vector of the Cloning Kit for mRNA Template (product number 6143, produced by Takara) to prepare a plasmid. The plasmid thus prepared was digested with restriction enzymes and separated by agarose gel electrophoresis. DNA fragments containing a T7 promoter, human α-globin 5'-UTR, viral protein ORF, human α-globin 3'-UTR, and poly(A) sequence were excised from the agarose gel, and the DNA was purified. Using the purified DNA as a template, mRNA was synthesized by in vitro transcription using a Takara IVTpro T7 mRNA Synthesis Kit (product number 6144, produced by Takara Bio Inc.). In the synthesis, 5-methoxyuridine (product number N-1093, produced by TriLink Biotechnologies) was used in place of UTP, and CleanCap Reagent AG (product number N-7113, produced by TriLink) was used as a material for the 5' cap. The synthesized IVT-mRNA was purified by using a Monarch RNA Cleanup Kit (produced by New England Biolabs), and the quality of the purified IVT-mRNA was examined by non-denaturing agarose gel electrophoresis using RNA High for Easy Electrophoresis (produced by DynaMarker Laboratory).

For virus exposure, the LASV-attenuated live vaccine candidate strain ML29 was used. ML29 is a genetic reassortant comprising an S segment derived from LASV (containing LASgpc gene and LASnp gene) and an L segment derived from Mopeia virus (MOPV), which is considered non-pathogenic to humans.

Using a NanoAssemblr^{®} Ignite microfluidic mixer (produced by Precision Nanosystems), LNP formulations (IVT-mRNA-LNPs) were prepared from GenVoy-ILM (produced by Precision Nanosystems) and IVT-mRNA produced in Fig. 6. The N/P ratio (the molar ratio of negatively charged phosphates on mRNA to cationic amines on ionizable lipids) was set to 4. The obtained samples were diluted with 1× Formulation Buffer 2 (produced by Precision Nanosystems), concentrated with Amicon Ultra Centrifugal Filters (produced by Millipore), and passed through a 0.2-µm filter for sterile filtration. The filtered mRNA-LNPs were stored at 4°C until use. The concentration of IVT-mRNA incorporated into LNPs was determined using a Quant-iT Ribogreen Assay Kit (produced by Thermo Fisher Scientific). LASgpc-mRNA-LNP or LASnp-mRNA-LNP each containing 2 µg of IVT-mRNA, or physiological saline was administered intramuscularly to CBA mice twice, 3 weeks apart. Four weeks after the second vaccination, a lethal dose of ML29 was inoculated intracerebrally to examine the protective effect against virus exposure. Fig. 7 shows the results. All mice that received physiological saline died within 8 days. In contrast, all mice that received LASgpc-mRNA-LNP survived, whereas 4 out of 5 mice that received LASnp-mRNA survived. These results showed that intramuscular administration of LASgpc-mRNA-LNP or LASnp-mRNA-LNP vaccines confers protective immunity against lethal virus exposure.

### Example 12: Verification of protective effect by intramuscular administration of mRNA-LNP mixed vaccine in CBA mice

Whether a synergistic effect could be obtained by combining an LASgpc-mRNA-LNP vaccine and LASnp-mRNA-LNP vaccine, as compared to the effect achieved by administering each vaccine alone, was verified.

LASgpc-mRNA-LNP containing 0.4 µg of LASgpc-mRNA, or LASnp-mRNA-LNP containing 0.4 µg of LASnp-mRNA, or a mixture of LASgpc-mRNA-LNP containing 0.2 µg of LASgpc-mRNA and LASnp-mRNA-LNP containing 0.2 µg of LASnp-mRNA, or physiological saline was intramuscularly administered to CBA mice twice, 3 weeks apart. Four weeks after the second administration, the mice were inoculated intracerebrally with a lethal dose of ML29 to examine the protective effect against virus exposure. Fig. 8 shows the results. All mice that received physiological saline died within 8 days. Further, 1 out of 5 mice died in the LASgpc-mRNA-LNP administration group, whereas 4 out of 5 mice died in the LASnp-mRNA-LNP administration group. In contrast, all the mice that were inoculated with the mixed vaccine of LASgpc-mRNA-LNP and LASnp-mRNA-LNP survived. Although 100% of the mice did not survive with the administration of either LASgpc-mRNA-LNP or LASnp-mRNA-LNP containing 0.4 µg of IVT-mRNA, 100% of the mice survived with the administration of a mixture of LASgpc-mRNA-LNP and LASnp-mRNA-LNP each containing half the amount (0.2 µg) thereof. These results show that a combination of LASgpc-mRNA-LNP and LASnp-mRNA-LNP provides a synergistic effect.

## Claims

1. A vaccine comprising lipid nanoparticles each containing mRNA encoding a glycoprotein precursor (GPC) of arenavirus or mRNA encoding a nucleoprotein (NP) of arenavirus, or both.

2. The vaccine according to claim 1, wherein the GPC and the NP are of the same or different origins and are derived from Lassa virus or lymphocytic choriomeningitis virus.

3. The vaccine according to claim 1 or 2, wherein the GPC and the NP are of a wild type.

4. The vaccine according to claim 1 or 2, comprising lipid nanoparticles in which the mRNA encoding the GPC of arenavirus and the mRNA encoding the NP of arenavirus are encapsulated together in the same nanoparticle or individually encapsulated in separate nanoparticles.

5. The vaccine according to claim 1 or 2, wherein the mRNA encoding the GPC of arenavirus or the mRNA encoding the NP of arenavirus further comprises a 5' cap structure, a 5' untranslated region (UTR), a 3' UTR, and a 3' polyadenylated tail.

6. The vaccine according to claim 4, wherein the 5' UTR and the 3' UTR are derived from β-globin or α-globin.

7. The vaccine according to claim 4, wherein the 3' polyadenylated tail is 200 nucleotides or less in length.

8. The vaccine according to claim 1 or 2, wherein uridine contained in the mRNA is 5-methoxyuridine.

9. The vaccine according to claim 1 or 2, wherein the lipid nanoparticles have a number average particle size of 70 nm to 130 nm.

10. The vaccine according to claim 1 or 2 for intravenous or intramuscular administration.

11. The vaccine according to claim 1 or 2 for use in administration to a subject at risk of arenavirus infection.

12. The vaccine according to claim 11, wherein the arenavirus is Lassa virus and/or lymphocytic choriomeningitis virus.

13. The vaccine according to claim 1 or 2, which is for use in administration in an amount such that the mRNA content per dose of the vaccine is 0.1 µg to 200 µg.

14. A method for preventing an arenavirus infection, comprising administering a vaccine comprising lipid nanoparticles containing mRNA encoding a glycoprotein precursor (GPC) of arenavirus or mRNA encoding a nucleoprotein (NP) of arenavirus, or both.

15. Use of lipid nanoparticles each containing mRNA encoding a glycoprotein precursor (GPC) of arenavirus or mRNA encoding a nucleoprotein (NP) of arenavirus, or both in the production of a vaccine.
